# EUROPEAN PATENT APPLICATION

(11) **EP 3 848 041 A1**
(43) Date of publication of application: **14.07.2021**
(21) Application number: 19857206.7
(22) Date of filing: 04.09.2019
(51) Int. Cl.: A61K 35/20, A23L 33/10, A61P 1/04, A61P 3/04, A61P 3/10, A61P 9/10, A61P 17/00, A61P 25/16, A61P 25/18, A61P 25/24, A61P 25/28, A61P 29/00, A61P 31/04, A61P 43/00, C12N 9/24

(54) **INFLAMMATORY CYTOKINE PRODUCTION INHIBITOR**

(30) Priority: 05.09.2018 JP 2018166062
(71) Applicant: Saisei Pharma Co., Ltd., Moriguchi-shi, Osaka 570-0012 (JP)
(72) Inventor: INUI, Toshio, Moriguchi-shi, Osaka 570-0011 (JP)
(74) Representative: Cabinet Beau de Loménie
(86) International application number: PCT/JP2019/034784
(87) International publication number: WO 2020/050318

(57) **Abstract**

Provided are inflammatory cytokine production inhibitors which inhibit the production of the inflammatory cytokines TNF-α and IL-1β and can be applied to drugs effective against inflammatory diseases caused by overproduction of these inflammatory cytokines, such as chronic inflammation (e.g., rheumatoid arthritis, ulcerative colitis) and Crohn's disease, as well as type 2 diabetes, depression, obesity, sepsis, atherosclerosis, dermatitis, dementia, schizophrenia, and Parkinson's disease, and also to foods and drinks such as health foods. The inflammatory cytokine production inhibitors contain as an active ingredient an enzyme-treated whey obtained by contacting whey with β-galactosidase. The enzyme-treated whey is obtained by further contacting with sialidase.

## Description

### TECHNICAL FIELD

The present invention relates to inflammatory cytokine production inhibitors and specifically to inflammatory cytokine production inhibitors containing as an active ingredient an enzyme-treated whey obtained by enzyme treatment of whey, as well as inflammatory cytokine production inhibitory foods and drinks.

### BACKGROUND ART

Inflammatory cytokines are substances which are produced from lymphocytes, macrophages, and other cells and which are involved in inflammatory responses associated with bacterial or viral infection, tumors, or histological damage. For example, inflammatory cytokines such as interleukin-1β (IL-1β), interleukin-6 (IL-6), and tumor necrosis factor (TNF-α) originally have purposeful functions such as activation of immune functions against invasion by pathogenic bacteria, but it is known that when continuously overproduced due to certain causes, they can cause a variety of inflammatory diseases such as rheumatoid arthritis, ulcerative colitis, Crohn's disease, type 2 diabetes, and obesity (especially insulin resistance).

From the standpoint of neuroinflammation, they are also known to cause depression, for example.

Thus, inflammation is a defensive response that occurs when foreign substances invade the body or when harmful stimuli act on the body, and the inflammatory response is known to possibly cause diseases such as atherosclerosis, neurological diseases, and cancers.

In this context, studies have been performed for development of drugs for inhibiting the production of the inflammatory cytokines such as IL-1β and TNF-α in the pathological conditions mentioned above. For example, in addition to the conventional anti-inflammatory agents such as ibuprofen and indomethacin, a variety of chemical substances have been proposed (for example, see Patent Literatures 1 to 3). However, the aforementioned diseases often follow a chronic course and may require prolonged treatment. Thus, it is particularly desirable to provide a safe compound without side effects. In such a situation, effective compounds have not yet appeared.

From the aforementioned standpoint, some studies (Patent Literatures 4 and 5) have proposed inflammatory cytokine production inhibitors which contain juice and/or extract of fruit or the iron-binding glycoprotein lactoferrin, rather than organic compounds, as an active ingredient mainly for the purpose of ensuring safety.

Meanwhile, in the course of cheese production, whey or milk whey is separated from curds and most part of the whey is disposed of. However, whey has been increasingly recognized as an excellent food because of its advantages, such as being high in protein and low in fat, having a high nutritional value, including inorganic nutrients such as milk-derived calcium and various vitamins such as B vitamins, being rapidly digestible, and promoting protein synthesis and insulin secretion. Recently, various techniques for effective utilization of whey have been proposed.

The present inventors have also made efforts to effectively utilize this whey and newly found that an enzyme-treated product obtained by treating and cutting the carbohydrate chains of whey proteins (glycoproteins) contained in whey with a specific enzyme, i.e., an enzyme-treated whey (Note: a whey treated with a specific enzyme) has an excellent effect of inhibiting inflammatory cytokine production. This finding has led to the completion of the present invention.

### CITATION LIST

### PATENT LITERATURE

Patent Literature 1: JP 2015-174850 A
Patent Literature 2: JP 2014-101329 A
Patent Literature 3: JP 2009-013106 A
Patent Literature 4: JP 2005-089304 A
Patent Literature 5: JP 2006-069995 A
Patent Literature 6: JP 2002-080387 A

### SUMMARY OF INVENTION

### TECHNICAL PROBLEM

Accordingly, the present invention aims to provide inflammatory cytokine production inhibitors which inhibit the production of the inflammatory cytokines TNF-α and IL-1β and can be applied to drugs effective against inflammatory diseases caused by overproduction of these inflammatory cytokines, such as rheumatoid arthritis, ulcerative colitis, Crohn's disease, and type 2 diabetes, and also to foods and drinks such as health foods.

### SOLUTION TO PROBLEM

To solve the above issue, the present invention may be provided as any of the following basic embodiments:
(1) an inflammatory cytokine production inhibitor, containing as an active ingredient an enzyme-treated whey obtained by contacting whey with β-galactosidase;
(2) the inflammatory cytokine production inhibitor according to the embodiment (1), wherein the enzyme-treated whey is obtained by further contacting with sialidase;
(3) the inflammatory cytokine production inhibitor according to the embodiment (1) or (2), wherein the inflammatory cytokine is interleukin-1β (IL-1β), interleukin-6 (IL-6), or tumor necrosis factor (TNF-α);
(4) the inflammatory cytokine production inhibitor according to any one of the embodiments (1) to (3), wherein the enzyme-treated whey contains 0.02 µg to 40 mg/kg/dose of proteins;
(5) a drug or a food or drink, containing the inflammatory cytokine production inhibitor according to any one of the embodiments (1) to (3);
(6) the drug or the food or drink according to the embodiment (5), wherein the drug or the food or drink is in an orally administrable or orally ingestible form;
(7) a method for preparing an enzyme-treated whey having an effect of inhibiting inflammatory cytokine production, the method including contacting whey with β-galactosidase to cleave a β-1,6-glycosidic bond of a protein in the whey;
(8) the method according to the embodiment (7), wherein the β-galactosidase is derived from *Escherichia coli* or bovine liver; and
(9) the method for preparing an enzyme-treated whey having an effect of inhibiting inflammatory cytokine production according to the embodiment (7) or (8), including further contacting with sialidase.

### ADVANTAGEOUS EFFECTS OF INVENTION

The present invention provides highly safe, orally administrable or orally ingestible inflammatory cytokine production inhibitors and inflammatory cytokine production inhibitory foods and drinks.

The inflammatory cytokine production inhibitors provided by the present invention inhibit overproduction of inflammatory cytokines such as interleukin-1β (IL-1β), interleukin-6 (IL-6), and tumor necrosis factor (TNF-α), for example. Thus, the inflammatory cytokine production inhibitors may serve as therapeutic agents effective against a variety of diseases caused by overproduction of these cytokines, such as chronic inflammation (e.g., rheumatoid arthritis, ulcerative colitis) and Crohn's disease, as well as type 2 diabetes, depression, obesity, sepsis, atherosclerosis, dermatitis, dementia, schizophrenia, and Parkinson's disease. Further, the inflammatory cytokine production inhibitors of the present invention have the advantage of providing daily healthcare effectively by daily oral ingestion of foods or drinks containing them.

### BRIEF DESCRIPTION OF DRAWINGS

Fig. 1 is a diagram showing the test schedule for analyzing the effect of an enzyme-treated whey (CWP) according to the present invention on lipopolysaccharide (LPS) responses in Example 2.
Fig. 2 is a graph showing the weight changes of test mice after LPS administration in Example 2.
Fig. 3 is a graph showing the weight changes in comparison of 5-day test group 1 (CWP-administered group) and control group 1 (saline-administered group) in Example 2.
Fig. 4 is a graph showing the changes in food consumption during the administration period in comparison of 5-day test group 1 (CWP-administered group) and control group 1 (saline-administered group) in Example 2.
Fig. 5 is a graph showing the food consumption during the 24-hour period after Day 5 administration in accordance with the administration schedule in comparison of test group 1 (CWP-administered group) and control group 1 (saline-administered group) in Example 2.
Fig. 6 is a graph showing the changes in blood TNF-α after LPS administration (Result 2 in Example 2).
Fig. 7 is a graph showing the changes in blood IL-1β after LPS administration (Result 2 in Example 2).

### DESCRIPTION OF EMBODIMENTS

As described above, the basic embodiments of the present invention provide inflammatory cytokine production inhibitors containing as an active ingredient an enzyme-treated whey obtained by contacting whey with β-galactosidase.

The whey used here is generally a pale yellow-green liquid that is obtained in a large amount as a by-product when curds are separated in the course of cheesemaking. However, the whey in the present invention is not limited thereto and those available as "whey" may also be used.

The milk as a material of cheesemaking is not limited to milk from bovines. The milk whey may be derived from milk of other mammals such as sheep, goats, and camels.

The bovines used here may be any bovine species, including, but not limited to, Holstein and Japanese Black cattle, and water buffalo.

Hereinafter, the term "milk whey" is referred to simply as "whey".

The enzyme-treated whey as the inflammatory cytokine production inhibitor provided by the present invention is an enzyme-treated whey obtained by contacting the aforementioned whey with β-galactosidase.

The β-galactosidase used here is not limited, and any well-known type of β-galactosidase can be used. Examples of such β-galactosidase include those derived from *Escherichia coli* or bovine liver. Preferred is β-galactosidase derived from *Escherichia coli.*

Examples of such commercially available β-galactosidase include catalog No. 072-04141 (derived from *Escherichia coli)* available from Fujifilm Wako Pure Chemical Corp., β-galactosidase (derived from *Escherichia coli)* available from Oriental Yeast Co., Ltd., and G1875 (derived from bovine liver) available from Sigma-Aldrich Co. LLC.

The enzyme-treated whey as the inflammatory cytokine production inhibitor provided by the present invention is such that whey is treated with such β-galactosidase to cleave the β-1,6-glycosidic bond of the protein in the whey, i.e., to cleave the carbohydrate moiety, thereby forming a treated product having a desired protein content, which exhibits an effect of inhibiting inflammatory cytokine production. As is clear from the EXAMPLES described later, whey itself without such enzyme treatment, i.e., enzyme-untreated whey, was found to have no effect of inhibiting inflammatory cytokine production.

In the present invention, in addition to the aforementioned treatment with β-galactosidase, the whey may be further treated with sialidase, a glycolytic enzyme that removes the sialic acid residues linked by an α-glycosidic bond from the carbohydrate chain ends of glycolipids.

Such sialidase is not limited, and any well-known type of sialidase can be used. Examples include those derived from *Clostridium perfringenes, Streptococcus* 6646K, *Vibrio cholerae,* and *Arthrobacter ureafaciens.*

Examples of commercially available sialidase include product numbers (Sigma Prod. Nos.) N2876, N2133, N2904, N3001, and N5631 all available from Sigma-Aldrich Co. LLC., code number 120052 available from Seikagaku Biobusiness Corp., and catalog # P0720L and P0720S both available from New England BioLabs.

The treatment (enzyme treatment) of contacting whey with β-galactosidase or with β-galactosidase and sialidase according to the present invention is preferably carried out by contacting with a sufficient amount of the enzyme(s) for a sufficient duration until the enzyme reaction(s) does not proceed substantially further.

The amount of the enzyme used for the above purpose may vary depending on the type of enzyme. When the β-galactosidase used is catalog No. 072-04141 available from Fujifilm Wako Pure Chemical Corp., for example, it is sufficient to use about 65 mU of the enzyme per 100 µL of whey. When the sialidase used is N2876 (product number) available from Sigma-Aldrich Co. LLC., for example, it is sufficient to use about 65 mU of the enzyme per 100 µL of whey. In these cases, it is sufficient that the enzyme treatment is performed for about 3 hours.

The enzyme treatment can be carried out by adding the enzyme(s) to whey in any vessel, optionally to which a buffer solution commonly used in this field has been added in order to control the total protein concentration in the whey. Examples of the buffer solution include saline, phosphate-buffered saline (SPB), and Ringer's solution.

The temperature of the enzyme treatment may be any temperature at which the enzyme exhibits its activity, and is usually around 37°C at which enzymes exhibit high activity.

The enzyme treatment is stopped by inactivating the enzyme(s) by heating (heat treatment). The heat treatment may be any treatment that can inactivate the enzyme(s). For example, it may be carried out by heating at around 60°C for about 10 minutes.

Of course, the enzyme treatment in the present invention may also be performed using an enzyme immobilized on a solid phase (immobilized enzyme) as generally known.

The enzyme-treated whey thus prepared may be used as it is, or may alternatively be prepared into a concentrated liquid by solvent evaporation or may be dried before use.

The drying may be carried out by a usual drying technique such as vacuum drying, freeze drying, or spray drying. Among these, freeze drying is preferably performed to form a dried product.

Thus, whey may be enzyme treated to obtain an enzyme-treated whey having a desired protein content.

The enzyme-treated whey provided by the present invention has an excellent effect in inhibiting the production of inflammatory cytokines such as IL-1β, IL-6, and TNF-α, and is useful as an inflammatory cytokine production inhibitor.

The inflammatory cytokine production inhibitors of the present invention may be administered in any manner depending on the purpose of administration, the type of disease, and the particular symptoms. The inhibitors may be in the dosage form of a tablet, a capsule, granules, powder, a powdered medicine, liquid, or other forms for direct administration or may be mixed into a food or drinking water for administration, and are desirably orally administered.

These dosage forms can be prepared by usual conventional methods. Appropriate additives such as dextrin, lactose, cornstarch, emulsifiers, antiseptics, vehicles, expanders, sweetening agents, flavors, and colorants may be incorporated as long as the advantageous effect of the present invention is not impaired.

The inflammatory cytokine production inhibitors in such cases may be administered at a dose such that the enzyme-treated whey contains 0.02 µg to 40 mg/kg/dose of proteins.

Moreover, the inflammatory cytokine production inhibitors of the present invention may also be used as foods or drinks. Examples of such foods or drinks include non-alcoholic drinks such as soft drinks and juices, alcoholic drinks, fermented drinks such as yogurt, hard sweets such as tablets and candies, chewable sweets such as gum and gummies, nutritional supplements containing, for example, vitamins, minerals, amino acids, or proteins, and other forms.

Examples of such foods or drinks functionally include foods for specified health uses ("TOKUHO"), foods with nutrient function claims, and foods with function claims.

The foregoing agents and foods and drinks have the ability to inhibit the production of inflammatory cytokines and can be very useful for the prevention, treatment, improvement, or relapse prevention of a variety of pathological conditions caused by overproduction of inflammatory cytokines.

Examples of such target diseases include a variety of diseases caused by overproduction of these cytokines, such as chronic inflammation (e.g., rheumatoid arthritis, ulcerative colitis) and Crohn's disease, as well as type 2 diabetes, sepsis, depression, obesity, sepsis, atherosclerosis, dermatitis, dementia, schizophrenia, and Parkinson's disease.

For the inflammatory cytokine production inhibitors of the present invention, the dose for achieving the ability to inhibit the production of inflammatory cytokines is not limited, and may vary in accordance with the purpose of administration, the type of disease, and the particular symptoms. For example, the amount may be adjusted so that 1 to 5000 mg, preferably 5 to 1000 mg, more preferably 10 to 200 mg, of the enzyme-treated whey can be ingested per day.

In other words, the inflammatory cytokine production inhibitors are preferably ingested at a dose such that the enzyme-treated whey contains 0.02 µg to 40 mg/kg/dose of proteins.

The enzyme-treated whey according to the present invention is obtained by enzyme treatment of daily ingestible whey, and thus is highly safe and causes no toxicity problem at the doses used in the present invention.

### EXAMPLES

The present invention is described in more detail below with reference to examples and experimental examples. These are mere examples and are not at all intended to limit the present invention.

### Example 1: Preparation of enzyme-treated whey

Crude curd-removed milk (whey obtained from General Incorporated Foundations Zao Dairy Center), which was prepared by treating bovine milk (cow's milk) to remove cheese components (curds), was centrifuged at 8000 rpm and 4°C for one hour, and the supernatant was collected while paying attention to the precipitates. To the supernatant was added an equal amount of distilled water (Mill-Q grade, the same grade is used hereinafter, unless otherwise indicated), and the mixture was dialyzed using a pencil scale module UF membrane (AIP-0013D available from Funakoshi Co., Ltd.). The dialysis was performed until the amount of the filtrate reached the amount of distilled water added. Thus, a curd-removed milk (whey) was obtained.

The protein concentration of the curd-removed milk (whey) was determined by absorbance measurement at a wavelength of 570 nm (with a calibration curve prepared for bovine serum albumin (BSA, A4503 available from Sigma-Aldrich Co. LLC.)).

The curd-removed milk (whey) obtained above was dispensed to have a protein content of 6 g. To the resulting curd-removed milk (whey) was added 4 g/6000 U of β-galactosidase (derived from *Escherichia coli,* available from Oriental Yeast Co., Ltd.) immobilized on a formyl resin (Formy-650M available from TOYOPEARL), and the mixture was incubated at 37°C for one hour. The incubation was followed by filtrating the reaction solution through a glass filter to separate the formyl resin. The formyl resin was washed with distilled water and stored for repetitive use. The filtrate was filter-sterilized using Labodisc (50CP020AS available from Advantec Toyo Kaisha, Ltd.). The sterilized filtrate was freeze-dried and powdered to obtain a storable powdery enzyme-treated whey.

### Example 2: Effect of enzyme-treated whey according to present invention on LPS responses

### <Method>

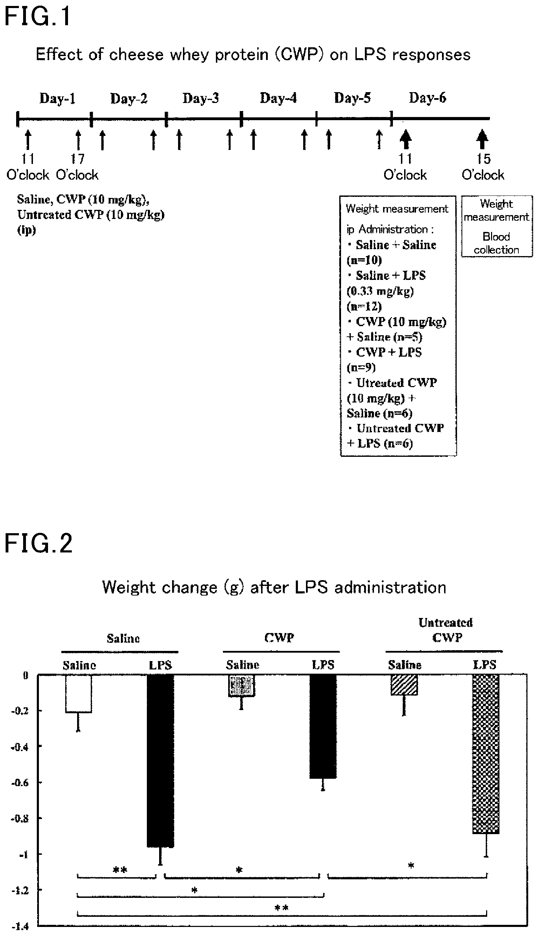

According to the test schedule shown in Fig. 1, the effect of the enzyme-treated whey (cheese whey protein, CWP) according to the present invention on LPS responses was analyzed.

As also used hereinafter (in the description and the drawings), the terms "CWP" and "untreated CWP" refer as follows:
CWP: enzyme-treated whey;
untreated CWP: enzyme-untreated whey.

### (1) Experimental animals

Used were 12- to 15-week-old male C57BL/6J mice (Charles River Laboratories Japan, Inc.).

### (2) Test schedule

The schedule was such that the test sample was administered intraperitoneally (ip) twice (at 11 o'clock and 17 o'clock) a day for five consecutive days.

Each experimental animal was weighed at the time of twice-daily administration, and the food consumption during the administration period and the food consumption during the 24-hour period after the 5-day administration were determined.

The administration was performed until 11 o'clock on Day 6 after the start of the test. At 15 o'clock on Day 6, body weight was measured and a blood sample was collected. The blood TNF-α and IL-1β concentrations were determined using the respective ELISA kits.

Statistical significance was tested using the Tukey-Kramer test.

### (3) Test sample-administered groups

The test sample-administered groups were as follows (n represents the number of animals used).
- Control group 1: Saline + Saline (n = 10)
- Control group 2: Saline + LPS (n = 12)
- Test group 1: CWP + Saline (n = 5)
- Test group 2: CWP + LPS (n = 9)
- Comparative test group 1: Untreated CWP + Saline (n = 6)
- Comparative test group 2: Untreated CWP + LPS (n = 6)

The amount of CWP or untreated CWP administered was 10 mg/kg, and the amount of LPS administered was 0.33 mg/kg.

### (4) Blood collection method

Blood was collected from the orbital artery into a tube. The tube contained 10 µL of 0.2 M EDTA and 0.1 mg of aprotinin.

### (5) Separation of plasma

The blood sample was centrifuged at 4°C and 3000 × g for four minutes to collect the plasma. The plasma was stored at -80°C until assay.

### (6) Method for measuring blood TNF-α and IL-1β

The measurements were performed using mouse Quantike TNF-α ELISA Kit (R&D Systems Inc.) for TNF-α and mouse Quantike IL-1β ELISA Kit (R&D Systems Inc.) for IL-1β in accordance with the instructions of the respective kits.

### <Results>

### Result 1: Weight change after LPS administration

Fig. 2 shows the weight changes after LPS administration.

LPS serves as an endotoxin to promote secretion of various inflammatory cytokines through TLR4 (Toll-like receptor 4)-mediated signaling pathways, thereby causing inflammatory responses.

As is clear from the results shown in the figure, weight reduction caused by LPS administration was observed. In the CWP-administered group, however, such weight reduction was significantly decreased. This demonstrates a decrease in the feeding deterrent effect associated with the increase in cytokine production caused by LPS.

In contrast, in the untreated CWP-administered group, no decrease in the weight reduction caused by LPS administration was observed. Thus, it is understood that CWP has a specific effect of inhibiting inflammatory cytokine production.

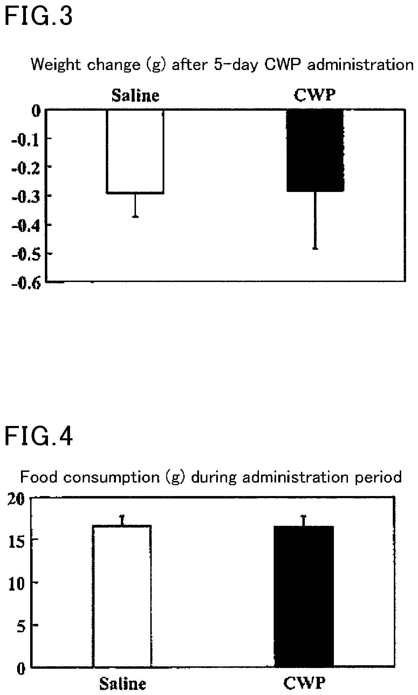

In Fig. 3, which shows the weight changes in comparison of 5-day test group 1 (CWP-administered group) and control group 1 (saline-administered group), the weight change of the CWP-administered group is similar to that of control group 1 (saline-administered group), and therefore it is understood that CWP was found to have no toxicity.

Similarly, in Fig. 4, which shows the changes in food consumption during the administration period in comparison of 5-day test group 1 (CWP-administered group) and control group 1 (saline-administered group), the food consumption of the CWP-administered group is similar to that of control group 1 (saline-administered group). From this, it is also understood that CWP was found to have no toxicity.

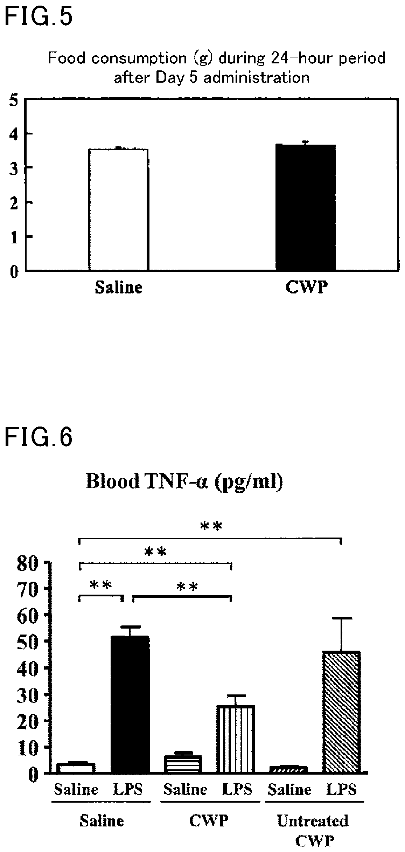

Also in Fig. 5, which shows the food consumption during the 24-hour period after Day 5 administration in accordance with the administration schedule in comparison of test group 1 (CWP-administered group) and control group 1 (saline-administered group), the CWP-administered group had food consumption similar to that of control group 1 (saline-administered group). This demonstrates the absence of any pathological conditions that cause feeding disorder in the test mice.

### Result 2: Changes in blood TNF-α and IL-1β after LPS administration

### (1) Change in blood TNF-α

Fig. 6 shows the changes in blood TNF-α after LPS administration.

As is clear from the results shown in the figure, LPS administration promoted the production of TNF-α as an inflammatory cytokine (a comparison of control group 1 and control group 2), but it is understood that when CWP according to the present invention is administered at the same time, the production of TNF-α as an inflammatory cytokine was significantly inhibited (a comparison of control group 1, control group 2, test group 1, and test group 2).

In contrast, in the untreated CWP-administered group, there was observed no effect of reducing the increased production of the inflammatory cytokine TNF-α caused by LPS administration. Thus, it is understood that CWP (enzyme-treated whey) according to the present invention has a specific effect of inhibiting inflammatory cytokine production.

### (2) Change in blood IL-1β

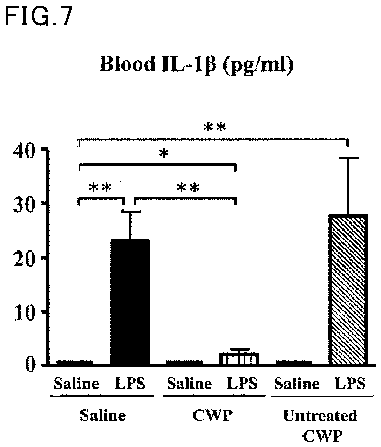

Fig. 7 shows the changes in blood IL-1β after LPS administration.

Similarly to the results of TNF-α described above, LPS administration promoted the production of IL-1β as an inflammatory cytokine (a comparison of control group 1 and control group 2), but it is understood that when CWP according to the present invention is administered at the same time, the production of IL-1β as an inflammatory cytokine was significantly inhibited (a comparison of control group 1, control group 2, test group 1, and test group 2).

In contrast, in the untreated CWP-administered group, there was observed no effect of reducing the increased production of the inflammatory cytokine IL-1β caused by LPS administration. Thus, it is understood that CWP (enzyme-treated whey) according to the present invention has a specific effect of inhibiting inflammatory cytokine production.

The above analysis results show that the specific enzyme-treated whey (cheese whey protein, CWP) provided by the present invention better exhibited an effect of inhibiting inflammatory cytokine production than the enzyme-untreated whey (untreated CWP) with no enzyme treatment, and therefore the enzyme-treated whey according to the present invention has an anti-inflammatory effect.

### INDUSTRIAL APPLICABILITY

The present invention provides inflammatory cytokine production inhibitors which are highly safe via oral administration or ingestion.

The inflammatory cytokine production inhibitors provided by the present invention inhibit overproduction of inflammatory cytokines such as interleukin-1β (IL-1β), interleukin-6 (IL-6), and tumor necrosis factor (TNF-α), for example. Thus, the inhibitors may serve as therapeutic agents effective against a variety of diseases caused by overproduction of these cytokines, such as chronic inflammation (e.g., rheumatoid arthritis, ulcerative colitis) and Crohn's disease, as well as type 2 diabetes, depression, obesity, sepsis, atherosclerosis, dermatitis, dementia, schizophrenia, and Parkinson's disease. Further, the inhibitors have the advantage of providing daily healthcare effectively by daily oral ingestion. Accordingly, the inhibitors have significant industrial applicability.

## Claims

1. An inflammatory cytokine production inhibitor, comprising as an active ingredient an enzyme-treated whey obtained by contacting whey with β-galactosidase.

2. The inflammatory cytokine production inhibitor according to claim 1,
wherein the enzyme-treated whey is obtained by further contacting with sialidase.

3. The inflammatory cytokine production inhibitor according to claim 1 or 2,
wherein the inflammatory cytokine is interleukin-1β (IL-1β), interleukin-6 (IL-6), or tumor necrosis factor (TNF-α).

4. The inflammatory cytokine production inhibitor according to any one of claims 1 to 3,
wherein the enzyme-treated whey contains 0.02 µg to 40 mg/kg/dose of proteins.

5. A drug or a food or drink, comprising the inflammatory cytokine production inhibitor according to any one of claims 1 to 3.

6. The drug or the food or drink according to claim 5,
wherein the drug or the food or drink is in an orally administrable or orally ingestible form.

7. A method for preparing an enzyme-treated whey having an effect of inhibiting inflammatory cytokine production, the method comprising
contacting whey with β-galactosidase to cleave a β-1,6-glycosidic bond of a protein in the whey.

8. The method according to claim 7,
wherein the β-galactosidase is derived from *Escherichia coli* or bovine liver.

9. The method for preparing an enzyme-treated whey having an effect of inhibiting inflammatory cytokine production according to claim 7 or 8, comprising further contacting with sialidase.
